# EUROPEAN PATENT SPECIFICATION

(11) **EP 0 855 922 B1**
(45) Date of publication and mention of the grant of the patent: **18.12.2002**
(21) Application number: 96935704.5
(22) Date of filing: 14.10.1996
(51) Int. Cl.: A61M 5/20, A61M 5/31

(54) **ARRANGEMENT IN ELECTRONICALLY CONTROLLED INJECTION DEVICES**
ANORDNUNG VON ELEKTRONISCH GESTEUERTEN INJEKTIONSVORRICHTUNGEN
AGENCEMENT DESTINE A DES DISPOSITIFS D'INJECTION A COMMANDE ELECTRONIQUE

(30) Priority: 20.10.1995 SE 9503685; 20.10.1995 US 5773 P
(43) Date of publication of application: 05.08.1998
(73) Proprietor: Pharmacia AB, 112 87 Stockholm (SE)
(72) Inventor: HJERTMAN, Birger, S-162 43 Vällingby (SE)
(74) Representative: Elwe, Magnus
(86) International application number: SE9601303
(87) International publication number: WO97014459

(56) References cited:
- EP-A- 0 285 679

## Description

The present invention relates to electronically controlled injection devices. More specifically, the invention relates to arrangements in electronically controlled injection devices, which facilitate a correct handling of the device when preparing it for the administering of one or more injections.

Electronically controlled injection devices have recently been developed and put on the market. Such devices are generally adapted to utilize an injection cartridge with one or more chambers, and comprise an electronic control unit which may be programmed to carry out the necessary operations for the administering of injections.

Examples of electronically controlled injection devices according to the prior art are described in EP-A-0 164 539 and EP-A-0 293 958. These references give a good overview of the state of the art in this field.

Electronically controlled injection devices have turned out to have a number of important advantages, especially in those cases where the patient has to administer the injections to himself, as is the case in the ambulatory treatment of diabetics with insulin. The dose to be administered may be set in advance by the physician, and may even be set at a different value depending on which time of the day the dose is to be administered. The timer will give a signal when it is time for administering a dose, and the programmed electric motor will ensure that the correct amount is administered. Through modem electronical engineering and integrated circuitry, the device may be made small enough to resemble a fountain pen, which can easily be carried in an inside jacket pocket or in a lady's purse.

However, there is still room for improvements in this field. It is desirable to let the operations for the administering comprise the zeroing of a counter and a display before and after the administering, the removal of entrapped air from the cartridge, the metering out of doses of a predetermined magnitude, and the giving of a signal when the cartridge is empty. It may also be desirable to provide a timer to give a signal at the proper time for administering an injection, as well as other functions. The device may often also comprise an electrical motor which, on a signal from the control unit, drives a piston rod for a predetermined distance, such that a predetermined dose is administered. Other functions are also possible. This especially applies to the preparation and readying of the injection device before the first administering is to be carried out from a freshly inserted injection cartridge. These preparation and readying steps include the mixing of components to reconstitute an injectable composition in the cartridge, when a multi-chamber cartridge is used, the removal of entrapped gas from the cartridge, and the zeroing of the counter and/or display unit in the control unit before the first administering. If a multi-chamber cartridge is used, the mixing of components to reconstitute an injectable preparation is also included in the readying steps.

A multi-chamber injection cartridge usually comprises two chambers within a cartridge barrel, which chambers are separated by a front piston. The front chamber contains a solid component of an injectable composition, and is closed at its front end by a closure which may be pierced by an injection needle or cannula. The rear chamber contains a liquid component of an injectable composition and is closed at its rear end by a rear piston. To prepare the cartridge for the administering of an injectable composition, the rear piston is moved forward. Through the essentially incompressible liquid in the rear chamber, the front piston will then also be moved forward, and will at a predetermined position activate a bypass connection, such that the liquid in the rear chamber will be urged over into the front chamber to be mixed with the solid component and form a solution or dispersion to be injected. Multi-chamber injection cartridges comprising more than two chambers are also known.

The design and function of single-chamber and multi-chamber injection cartridges are well-known to those skilled in the art; and need not be described here in more detail.

When an injection device is to be readied for the administering of injections, an injection cartridge is first positioned and secured in a predetermined location in the device, and a piston rod is brought into contact with the rear face of the rear piston. By advancement of the piston rod forward, the cartridge is then readied for administering, as is described in the foregoing, to reconstitute the injectable composition. During the reconstitution step, an injection needle may be inserted through the front end closure of the cartridge, to afford a connection with the outer atmosphere and prevent the build-up of an overpressure in the cartridge, but this is not strictly necessary at this stage of the process.

The reconstitution of the injectable composition must often be carried out under mild conditions, so that sensitive compositions, such as growth hormones, are not unduly decomposed. For this, the cartridge should be held vertically with its front end pointing upward, and the liquid component should be made to flow calmly into the front chamber without any shaking or agitation to aid in the dissolution of the solid component. Other reconstitution positions , such as vertical with the front end pointing downwards or horizontal, may be utilized, depending on e.g. distribution of the solids or by-pass design and location.

Generally when mixing components it may also be desirable to secure wetting of all solids and all wall surfaces or to impose a certain agitation by turning or rotating the device before subsequent steps in the administration scheme.

The next step in the readying of the injection device is the removal of bubbles of air and other gases from the cartridge. This is an important step, even though the amount of enclosed gas in modern injection cartridges is usually not sufficient to cause a health hazard of air embolism if it is accidentally injected. However, as the entrapped gases are compressible, they serve as a buffer, which may give rise to inaccuracies in the metering out of the liquid composition.

The removal of air and other gases is usually carried out in the conventional way: After having inserted an injection needle through the front closure of the cartridge (if this has not been done previously), the user holds the injection device vertically with the needle pointing upward, and advances the piston rod forward until no gas, but only liquid comes out through the needle.

After the gas has been expelled from the cartridge, the metering scale and its display is set at zero or some other desired value before the administering of the injectable composition is started.

In electronically controlled injection devices, the reconstitution of the injectable composition, the removal of enclosed air and the zeroing of the metering scale are usually carried out automatically when the user pushes a start button or the like.

From what has been stated above, it follows that the process of readying an injection device for the administering of injections is a rather delicate process, which demands considerable care from the user. However, this is often forgotten by the user, who usually has no medical training. Thus, the reconstitution of the injectable composition is often carried out with the cartridge in any arbitrary orientation, which may lead to an undesired decomposition of a sensitive composition. The removal of air may be forgotten completely, or may be carried out with the needle pointing in an unsuitable direction, which may lead to an ineffective removal and to spillage of the preparation.

Also during the administration process the device orientation may be critical. Some injection positions may be unsuitable and indicative of improper use. Inadvertent administration of multiple doses may take place unless the device has been moved or turned between two consecutive injections.

Through the present invention, the above shortcomings are eliminated.

According to the present invention, there is provided an injection device and a method for its operation for the administering of one or more injections from an injection cartridge which is arranged in a holder device and at its rear end is provided with a piston which be means of a piston rod may be moved forward, the readying of the device for administering after the positioning of said cartridge and the subsequent administering therefrom being controlled by an electronic control unit. The control unit comprises a position sensor which emits signals indicative of device orientation, which govern the control unit in such a way that during the readying of the device, forward movement of the piston rod is only made possible when the longitudinal axis of the injection cartridge is oriented in a predetermined direction.

Administration devices using sensors are known although not fro the present purposes. The EP 285679 reference discloses a sensor for detection of a syringe rod in relation to a syring holder for the purpose of monitoring the rod path during its squeezing. The sensor is not indicative for the orientation of the device as such.

Preferably, this predetermined direction is essentially vertical, with the front end of the cartridge, from which the administering is to take place, pointing upward. However, an essentially vertical direction with the front end of the cartridge pointing downward is also possible.

In a preferred embodiment, the movement of the piston rod is effected by means of an electric motor, which is controlled by the control unit.

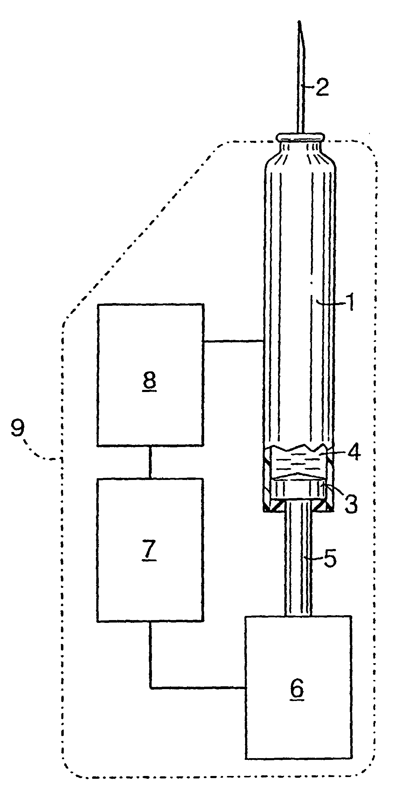

In a further preferred embodiment, the injection cartridge is a multi-chamber injection cartridge. The readying of the device for administering one or more injections comprises the reconstitution of an injectable composition and the removal of gases from said composition, and these processes, through the control by the position sensor, are made possible only with the logitudinal axis of the cartridge in an essentially vertical orientation with the front end of the cartridge pointing upward.
In the drawing, the arrangement of the invention is shown schematically. The injection device comprises an injection cartridge 1, which may be of the single-chamber or multi-chamber type. Preferably, the cartridge is of the dual-chamber type. The cartridge is provided with a needle or cannula 2 at its front end and with a rear piston 3 at its rear end. The cartridge contains a liquid component 4, which may fill the cartridge completely in the case of a single-chamber cartridge, or may fill a rear chamber in the case of a multi-chamber cartridge. The rear piston 3 is connected to a piston rod 5, which is actuated by an electric motor 6. The actuation of the motor 6 is governed by signals from the control unit 7.

The position or attitude sensor 8 is mechanically connected to the cartridge 1 in such a way that it senses the orientation of the cartridge 1 and sends corresponding signals to the control unit 7. All the elements of the injection device are arranged in a suitably shaped housing or holder device, which is indicated by the dashed line 9.

The injection device further comprises other elements for fulfilling other functions as desired, such as elements for timing, metering and display. Such elements are known to those skilled in the art, and are not shown in the drawing.

Position or attitude sensors of the type used in the present invention are known and may be of different types. It is also known to incorporate such sensors in an integrated circuit, for instance from the space and missile technology. With knowledge of the present invention, there will be no difficulty for a person skilled in the art to provide a suitable position sensor and install it properly in an electronic injection device.

For example, attitude sensors of this type are available on the market as "Non-Mercury Tip-Over Switches" from PEWATRON AG, Wallisellen/Zurich, Switzerland. These switches are available with different operating angles, i.e. the angle which the switch should be tilted from its vertical axis to switch from a closed to an open circuit. Other types of sensors include the well-known mercury switches. These are less suitable, however, as it is desirable nowadays to get away from the use of mercury as much as possible in all applications, because of its toxic properties.
With reference to the drawing, an example of the function of an injection device of the invention is as follows:

A fresh injection cartridge 1 is placed and secured in a predetermined location in the housing 9, and a needle 2 is inserted through the closure at its front end. The control unit 7 is then actuated to send a signal to the electrical motor 6 to start the readying of the cartridge 1 for the administering of injections.

In a first stage, the piston rod 5 is advanced to get into contact with the rear face of the rear piston 3. The piston 3 will then be urged further forward for the reconstitution of an injectable composition in the cartridge 1, as has been described in the foregoing. When the reconsititution of the composition is complete, the piston rod has been moved forward a given distance, which is sensed by the control unit 7 through signals from the motor 6. The motor usually then stops, as the expulsion of gas from the cartridge does not necessarily have to take place immediately after the reconstitution step.

When air or other gases are to be expelled from the cartridge, the user actuates the motor 6 through the control unit 7. The motor 6 is then kept running and the piston rod 5 is advanced as long as gas is expelled through the needle 2, and the movement of the piston rod 5 is stopped immediately when liquid starts to flow out from the needle 2. All gaseous components should now have been expelled from the cartridge 1.

After the zeroing of a metering scale and/or display (not shown), the device is ready for administering one or more injections. The dose to be injected may be set at this time, or it may already have been programmed into the control unit 7.

During the whole readying process described above, the position or attitude sensor 8 is in mechanical connection with the cartridge 1 and senses its orientation, usually via the housing 9. It is only when the cartridge is in a predetermined orientation, and preferably then an essentially vertical orientation with the needle pointing upward, that the signals from the position sensor 8 will make the control unit 7 send signals to the motor 6 which actuate the motor to move the piston rod 5 forward. Any important deviation from the vertical position will make the position sensor send a signal to the control unit 7 to make it stop the motor 6 and interrupt the readying process. In this way, it is prevented that the readying process takes place with the injection cartridge in an incorrect orientation. The disadvantages mentioned in the foregoing are thereby eliminated.

As stated above, an embodiment is also possible wherein the readying of the device for injection is carried out with the injection cartridge oriented in an essentially vertical direction, but with the front end of the cartridge pointing downward. The front end of the cartridge should be closed. In this embodiment, an overpressure generated when the solution is compressed by means of the piston rod 5 will open a pressure-sensitive valve in the rear piston, such that the air is expelled through the rear end of the cartridge. After the air has been removed from the cartridge, a needle may be attached to the front end of the cartridge, so that an injection may be administered.

After the device has been readied for injection, the control unit 7 will send a signal to the position or attitude sensor 8 such that the sensor is inactivated. This means that the device can now be used in any orientation when injections are to be administered.

The design and assembly of the elements in the device of the present invention is within the competence of a person skilled in the art, once said person has got an understanding of the inventive idea. The various elements are commercially available, or can be manufactured in ways known per se. Also the selection of suitable materials will not pose any great problems to one skilled in the art.

Through the present invention, there has been provided a new arrangement in electronically controlled injection devices, where a number of shortcomings associated with prior art devices have been largely eliminated. This arrangement will make it easier to avoid errors in the handling of electronically controlled injection devices.

It is to be noted that the description of the invention in the present specification and drawing only serves to exemplify the invention, and not to restrict it in any way. Variations and modifications of the embodiments disclosed are possible without departing from the scope of the claims. It is for example not necessary that the signals from the position sensor are used by a control unit for actuation of a motor but they may equally well be used to trigger an alarm or to signal e.g. a warning or instruction message to the operator. Similarly the position switch may not only be used during readying of the device but also during injection for sensing improper positions or proper turning of the device between injections. For the latter purpose the device should include means for registration of a sequence of positions, which is also of value to monitor an agitation step when desired.

## Claims

1. A portable or hand-held injection device comprising a container (1) for a preparation (4), or components for a preparation, an outlet (2) for the preparation and displacing means (3,5,6) at least able to displace the preparation through the outlet, **characterized in that** the device further comprises a position sensor (8) designed to emit one or more position signals indicative of its orientation and a control unit (7) designed to receive the position signals and issue one or more operation signals for the device, the operation signals being one or more messages and/or one or more control signals for the displacing means.

2. The device of claim 1, **characterized in that** the container (1) may contain gas and that the operation signals are designed to prevent displacing means (3,5,6) activation when device orientation is in first predetermined positions unsuitable for deaeration and to allow displacing means activation when device orientation is in second predetermined positions suitable for deaeration.

3. The device of claim 1, **characterized in that** the container (1) comprises at least two compartments for components of the preparation (4), that the displacing means (3,5,6) are operative to mix contents of at least two compartments and that the operation signals are designed to prevent displacing means activation when device orientation is in first predetermined positions unsuitable for mixing and to allow displacing means activation when device orientation is in second predetermined positions suitable for mixing.

4. The device of claim 3, **characterized in that** the container (1) is a dual or multi-chamber cartridge with at least one movable piston separating the compartments and a by-pass section for overflow of compartment content past the piston.

5. The device of claim 4, **characterized in that** the orientation suitable for mixing is with the outlet (2) pointing upwards, preferably substantially vertical.

6. The device of claim 1, **characterized in that** the control unit (7) is designed to register a change in position sensor (8) signals.

7. The device of claim 6, **characterized in that** the operation signals are designed to prevent displacing means (3,5,6) activation for ejection of preparation (4) through the outlet (2) unless the device has been turned in a predetermined manner.

8. The device of claim 6, **characterized in that** the operation signals are designed to prevent repeated displacing means (3,5,6) activation for ejection of preparation through the outlet (2) unless the device has been turned in a predetermined manner between the repeated displacement means activations.

9. The device of claim 1, **characterized in that** the operation signals are designed to prevent displacing means (3,5,6) activation for ejection of preparation through the outlet (2) when device orientation is in first predetermined positions unsuitable for administration and to allow displacing means activation for ejection of preparation through the outlet when device orientation is in second predetermined positions suitable for administration.

10. The device of claim 1, **characterized in that** the messages are one or more alarms or instructions detectable by an operator, for guidance of the operator to the actions stated.

11. The device of claim 1, **characterized in that** the displacing means (3,5,6) are activated by actuating means (6) and that the one or more operation signals are one or more control signals for the actuating means.

12. The device of claim 11, **characterized in that** the actuating means comprises electric motor means (6) for the displacement means (3,5).

13. The device of claim 1, **characterized in that** the displacing means (3,5,6) incorporates arming means, valve means or fluid control means and that the the one or more operation signals are one or more control signals for said means.

14. The device of any one of claims 1 to 13, **characterized in that** the control unit (7) is arranged to allow activation of the displacing means (3,5,6) with the device in any direction during injection following activation of the displacing means (3,5,6) for readying.

15. A method for operation of an injection device for the administering of one or more injections from an injection cartridge (1) which is arranged in a holder device (9) and at its rear end is provided with a piston (3) which by means of a piston rod (5) may be displaced forwards, and wherein the readying of the device for the administering after the positioning of said cartridge, and the subsequent administering therefrom are controlled by an electronic control unit (7) comprising a sensor, **characterized in that** the sensor is a position sensor (8) and that the method comprises the steps of a) extracting from the position sensor a signal indicative of the device orientation, b) feeding the signal to the control unit (7), and c) operating the control unit in such a way that during the readying step for the device, forward movement of the piston rod (5) is made possible only when the longitudinal axis of the injection cartridge (1) is oriented in a predetermined direction.

16. The method according to claim 15, **characterized in** the step of moving the piston rod (5) is effected by means of an electric motor (6), which is controlled by said control unit (7).

17. The method according to claim 15, **characterized in that** said readying step of the device comprises the step of removing gas from said injection cartridge (1).

18. The method according to any one of claims 15, **characterized in that** said cartridge (1) is a multi-chamber cartridge and **in that** said readying step also comprises a reconstitution step of an injectable composition (3).

19. The method according to claim 18, **characterized in that** said readying step of the device is made possible only with the longitudinal axis of the cartridge (1) in an essentially vertical orientation with its front end, from which the administering is to take place, pointing upward.

20. The method according to claim 18, **characterized in that** said readying step of the device is made possible only with the longitudinal axis of the injection cartridge (1) in an essentially vertical orientation, with the front end of the cartridge pointing downwards.

21. The method according to claim 15, **characterized in that** the operating step comprises the step of registering a change in position sensor (8) signals.

22. The method according to claim 21, **characterized in that** the operating step further comprises the step of preventing administering unless the device has been turned in a predetermined manner.

23. The method according to claim 21, **characterized in that** the operating step further comprises the step of preventing repeated administrations unless the device has been turned in a predetermined manner between the repeated aministrations.

24. The method according to claim 15, **characterized in that** the operating step further comprises the steps of preventing administration when device orientation is in first predetermined positions unsuitable for administration and allowing administration when device orientation is in second predetermined positions suitable for administration.

25. The method according to claim 15, **characterized in that** the operating step includes the step of issuing one or more messages and/or performing one or more displacements forwards of the piston (3).

26. The method according to claim 25, **characterized in that** the messages issued are one or more alarms or instructions detectable by an operator, for guidance of the operator to the actions stated.

27. The method according to claim 25, **characterized in that** the device comprises actuating means (6) and that the displacements are made by activation of the actuating means.

28. The method according to claim 27, **characterized in that** the actuating means activated are electric motor means (6).

29. The method according to claim 15, **characterized in** the step of allowing administration in any orientation after the readying step.

30. The method according to any one of claims 15 to 29, **characterized in that**, after the readying step, administration takes place by further displacement of the piston (3).

## Patentansprüche

1. Tragbare oder handgehaltene Injektionsvorrichtung mit einem Behälter (1) für eine Zubereitung (4) oder Bestandteile für eine Zubereitung, einem Auslass (2) für die Zubereitung und einer Verschiebeeinrichtung (3, 5, 6), die die Zubereitung zumindest durch den Auslass zu verschieben vermag, **dadurch gekennzeichnet, dass** die Vorrichtung ferner einen Positionssensor (8), der zum Abgeben einer oder mehrerer seine Orientierung angebender Positionssignale ausgeführt ist, und eine Steuereinheit (7) umfasst, die zum Empfangen der Positionssignale und Abgeben einer oder mehrerer Betriebssignale für die Vorrichtung ausgeführt ist, wobei die Betriebssignale eine oder mehrere Nachrichten und/oder ein oder mehrere Steuersignale für die Verschiebeeinrichtung sind.

2. Vorrichtung nach Anspruch 1, **dadurch gekennzeichnet, dass** der Behälter (1) Gas enthalten kann und dass die Betriebssignale dazu ausgeführt sind, eine Aktivierung der Verschiebeeinrichtung (3, 5, 6) zu verhindern, wenn die Vorrichtungsorientierung zur Entlüftung ungeeignete erste vorbestimmte Stellungen einnimmt, und eine Aktivierung der Verschiebeeinrichtung erlaubt, wenn die Vorrichtungsorientierung zur Entlüftung geeignete zweite vorbestimmte Stellungen einnimmt.

3. Vorrichtung nach Anspruch 1, **dadurch gekennzeichnet, dass** der Behälter (1) wenigstens zwei Abteile für Bestandteile der Zubereitung (4) umfasst, dass die Verschiebeeinrichtung (3, 5, 6) ein Vermischen der Inhalte von zumindest zwei Abteilen bewirkt, und dass die Betriebssignale dazu ausgeführt sind, eine Aktivierung der Verschiebeeinrichtung zu verhindern, wenn die Vorrichtungsorientierung zum Vermischen ungeeignete erste vorbestimmte Stellungen einnimmt, und eine Aktivierung der Verschiebeeinrichtung zu erlauben, wenn die Vorrichtungsorientierung zum Vermischen geeignete zweite vorbestimmte Stellungen einnimmt.

4. Vorrichtung nach Anspruch 3, **dadurch gekennzeichnet, dass** der Behälter (1) eine Doppel- oder Mehrfachkammerpatrone mit wenigstens einem die Abteile trennenden beweglichen Kolben und einem Bypassabschnitt zum Überströmen von Abteilinhalt am Kolben vorbei ist.

5. Vorrichtung nach Anspruch 4, **dadurch gekennzeichnet, dass** die Orientierung, die zum Vermischen geeignet ist, mit dem Auslass (2) nach oben zeigt, vorzugsweise im wesentlichen vertikal.

6. Vorrichtung nach Anspruch 1, **dadurch gekennzeichnet, dass** die Steuereinheit (7) dazu ausgeführt ist, eine Veränderung der Signale des Positionssensors (8) zu registrieren.

7. Vorrichtung nach Anspruch 6, **dadurch gekennzeichnet, dass** die Betriebssignale dazu ausgeführt sind, eine Aktivierung der Verschiebeeinrichtung (3, 5, 6) zum Ausstoßen von Zubereitung (4) durch den Auslass (2) zu verhindern, bis die Vorrichtung in einer vorbestimmten Weise gedreht worden ist.

8. Vorrichtung nach Anspruch 6, **dadurch gekennzeichnet, dass** die Betriebssignale dazu ausgeführt sind, eine wiederholte Betätigung der Verschiebeeinrichtung (3, 5, 6) zum Ausstoßen von Zubereitung durch den Auslass (2) zu verhindern, bis die Vorrichtung auf eine vorbestimmte Weise zwischen den wiederholten Betätigungen der Verschiebeeinrichtung gedreht worden ist.

9. Vorrichtung nach Anspruch 1, **dadurch gekennzeichnet, dass** die Betriebssignale dazu ausgeführt sind, eine Aktivierung der Verschiebeeinrichtung (3, 5, 6) zum Ausstoßen von Zubereitung durch den Auslass (2) zu verhindern, wenn die Vorrichtungsorientierung zur Verabreichung ungeeignete erste vorbestimmte Stellungen einnimmt, und eine Aktivierung der Verschiebeeinrichtung zum Ausstoßen von Zubereitung durch den Auslass zu erlauben, wenn die Vorrichtungsausrichtung zur Verabreichung geeignete zweite vorbestimmte Stellungen einnimmt.

10. Vorrichtung nach Anspruch 1, **dadurch gekennzeichnet, dass** die Nachrichten ein oder mehrere von einem Bediener feststellbare Alarme oder Anweisungen zum Leiten des Bedieners zu den angegebenen Aktionen sind.

11. Vorrichtung nach Anspruch 1, **dadurch gekennzeichnet, dass** die Verschiebeeinrichtung (3, 5, 6) durch Betätigungsmittel (6) aktiviert wird, und dass das eine oder die mehreren Betriebssignal(e) ein oder mehrere Steuersignale(e) für Betätigungsmittel sind.

12. Vorrichtung nach Anspruch 11, **dadurch gekennzeichnet, dass** die Betätigungsmittel elektrische Motormittel (6) für die Verschiebeeinrichtung (3, 5) umfassen.

13. Vorrichtung nach Anspruch 1, **dadurch gekennzeichnet, dass** die Verschiebeeinrichtung (3, 5, 6) Lademittel, Ventilmittel oder Fluidsteuermittel umfasst, und dass das eine oder die mehrere Betriebssignal(e) ein oder mehrere Steuersignale(e) für diese Mittel sind.

14. Vorrichtung nach einem der Ansprüche 1 bis 13, **dadurch gekennzeichnet, dass** die Steuereinheit (7) dazu ausgeführt ist, eine Aktivierung der Verschiebeeinrichtung (3, 5, 6) in jeder Ausrichtung der Vorrichtung während einer Injektion im Anschluss an eine Betätigung der Verschiebeeinrichtung (3, 5, 6) zum Fertigstellen zu erlauben.

15. Verfahren zum Betreiben einer Injektionsvorrichtung zum Verabreichen einer oder mehrerer Injektionen aus einer Injektionspatrone (1), die in einer Halteeinrichtung (9) angeordnet ist und an ihrem hinteren Ende mit einem Kolben (3) versehen ist, der mittels einer Kolbenstange (5) nach vorne verschoben werden kann, bei dem das Fertigstellen der Vorrichtung zum Verabreichen nach dem Positionieren der Patrone und das anschließende Verabreichen daraus durch eine elektronische Steuereinheit (7) mit einem Sensor gesteuert ist, durch **gekennzeichnet**, dass der Sensor ein Positionssensor (8) ist und dass das Verfahren die Schritte des a) Gewinnen eines die Vorrichtungsorientierung angebenden Signals aus dem Positionssensor, b) Zuführen des Signals zu der Steuereinheit (7), und c) Betreiben der Steuereinheit auf solche Weise umfasst, dass während des Fertigstellungsschrittes der Vorrichtung eine Vorwärtsbewegung der Kolbenstange (5) nur dann ermöglicht wird, wenn die Längsachse der Injektionspatrone (1) in einer vorbestimmten Richtung orientiert ist.

16. Verfahren nach Anspruch 15, **dadurch gekennzeichnet, dass** der Schritt des Bewegens der Kolbenstange (5) mittels eines Elektromotors (6) bewirkt wird, der von der Steuereinheit (7) gesteuert wird.

17. Verfahren nach Anspruch 15, **dadurch gekennzeichnet, dass** der Fertigstellungsschritt der Vorrichtung den Schritt des Entfernens von Gas aus der Injektionspatrone (1) umfasst.

18. Verfahren nach einem der Ansprüche 15 bis 17, **dadurch gekennzeichnet, dass** die Patrone (1) eine Mehrkammerpatrone ist und dass der Fertigstellungsschritt auch einen Wiederherstellungsschritt einer injizierbaren Lösung (3) umfasst.

19. Verfahren nach Anspruch 18, **dadurch gekennzeichnet, dass** der Fertigstellungsschritt der Vorrichtung nur ermöglicht wird, wenn die Längsachse der Patrone (1) sich in einer im wesentlichen vertikalen Ausrichtung befindet und ihr vorderes Ende, aus dem die Verabreichung stattfindet, nach oben weist.

20. Verfahren nach Anspruch 18, **dadurch gekennzeichnet, dass** der Fertigstellungsschritt der Vorrichtung nur ermöglicht wird, wenn sich die Längsachse der Injektionspatrone (1) in einer im wesentlichen vertikalen Ausrichtung befindet und das vordere Ende der Patrone nach unten weist.

21. Verfahren nach Anspruch 15, **dadurch gekennzeichnet, dass** der Betriebsschritt den Schritt des Registrierens einer Änderung von Signalen des Positionssensors (8) umfasst.

22. Verfahren nach Anspruch 21, **dadurch gekennzeichnet, dass** der Betriebsschritt ferner den Schritt des Verhinderns des Verabreichens umfasst, bis die Vorrichtung in einer vorbestimmten Weise gedreht worden ist.

23. Verfahren nach Anspruch 21, **dadurch gekennzeichnet, dass** der Betriebsschritt ferner den Schritt des Verhinderns wiederholten Verabreichens umfasst, bis die Vorrichtung zwischen den wiederholten Verabreichungen in einer vorbestimmten Weise gedreht worden ist.

24. Verfahren nach Anspruch 15, **dadurch gekennzeichnet, dass** der Betriebsschritt ferner die Schritte des Verhinderns einer Verabreichung, wenn die Vorrichtungsorientierung zum Verabreichen ungeeignete erste vorbestimmte Stellungen einnimmt, und des Erlaubens einer Verabreichung umfasst, wenn die Vorrichtungsorientierung zum Verabreichen geeignete zweite vorbestimmte Stellungen einnimmt.

25. Verfahren nach Anspruch 15, **dadurch gekennzeichnet, dass** der Betriebsschritt den Schritt des Ausgebens einer oder mehrerer Nachrichten und/oder des Ausführens einer oder mehrerer Vorwärtsverschiebungen des Kolbens (3) umfasst.

26. Verfahren nach Anspruch 25, **dadurch gekennzeichnet, dass** die ausgegebenen Nachrichten ein oder mehrere von einem Bediener wahrnehmbare Alarme oder Anweisungen zum Leiten des Bedieners zu den angegebenen Aktionen sind.

27. Verfahren nach Anspruch 25, **dadurch gekennzeichnet, dass** die Vorrichtung Betätigungsmittel (6) umfasst und dass die Verschiebungen durch eine Aktivierung der Betätigungsmittel bewirkt werden.

28. Verfahren nach Anspruch 27, **dadurch gekennzeichnet, dass** die aktivierten Betätigungsmittel Elektromotormittel (6) sind.

29. Verfahren nach Anspruch 15, **gekennzeichnet durch** den Schritt des Erlaubens einer Verabreichung in jeder Orientierung nach dem Fertigstellungsschritt.

30. Verfahren nach einem der Ansprüche 15 bis 29, **dadurch gekennzeichnet, dass** nach dem Fertigstellungsschritt eine Verabreichung durch eine weitere Verschiebung des Kolbens (3) stattfindet.

## Revendications

1. Un dispositif d'injection portable ou à tenir à la main comportant un conteneur (1) pour une préparation (4) ou des composants pour une préparation, un orifice de sortie (2) pour la préparation et un système de déplacement (3, 5, 6) capable au moins de déplacer la préparation à travers l'orifice de sortie, **caractérisé par le fait que** le dispositif comporte en outre un détecteur (8) conçu pour émettre un ou plusieurs signaux de position indicateurs de son orientation et un équipement de commande (7) pour recevoir les signaux et lancer un ou plusieurs signaux de fonctionnement pour le dispositif, les signaux de fonctionnement étant un ou plusieurs messages et/ou des signaux de commande pour le système de déplacement.

2. Le dispositif de la revendication 1, **caractérisé par le fait que** le conteneur (1) peut contenir du gaz et que les signaux de fonctionnement sont conçus pour empêcher une activation du système (3, 5, 6) de déplacement lorsque l'orientation du dispositif est dans une première série de positions prédéterminées et inappropriées pour une évacuation de l'air et pour permettre une activation du système de déplacement lorsque l'orientation du dispositif est dans une seconde série de positions prédéterminées et appropriées pour une évacuation de l'air.

3. Le dispositif de la revendication 1, **caractérisé par le fait que** le conteneur (1) comporte au moins deux compartiments pour des composants de la préparation (4), que le système (3, 5, 6) de déplacement est à même d'effectuer le mélange des contenus d'au moins deux compartiments et que les signaux de fonctionnement sont conçus pour empêcher une activation du système (3, 5, 6) de déplacement lorsque l'orientation du dispositif est dans une première série de positions prédéterminées et inappropriées pour un mélange et pour permettre une activation du système de déplacement lorsque l'orientation du dispositif est dans une seconde série de positions prédéterminées et appropriées pour un mélange.

4. Le dispositif de la revendication 3, **caractérisé par le fait que** le conteneur (1) est une cartouche à compartiment double ou multiple avec au moins un piston déplaçable séparant les compartiments et une section by-pass pour le déversement du contenu du compartiment au passage du piston.

5. Le dispositif de la revendication 4, **caractérisé par le fait que** l'orientation appropriée pour le mélange est celle avec l'orifice de sortie (2) orientée vers le haut, de préférence essentiellement verticalement.

6. Le dispositif de la revendication 1, **caractérisé par le fait que** l'équipement de commande (7) est conçu pour enregistrer un changement des signaux de détecteur (8) de position.

7. Le dispositif de la revendication 6, **caractérisé par le fait que** les signaux de fonctionnement sont conçus pour empêcher l'activation du système (3, 5, 6) de déplacement en vue d'une éjection de la préparation (4) à travers l'orifice de sortie (2) à moins que le dispositif n'ait été orienté d'une manière prédéterminée.

8. Le dispositif de la revendication 6, **caractérisé par le fait que** les signaux de fonctionnement sont conçus pour empêcher une répétition de l'activation du système (3, 5, 6) de déplacement en vue d'une éjection de la préparation à travers l'orifice de sortie (2) à moins que le dispositif n'ait été orienté d'une manière prédéterminée entre les activations répétées du système de déplacement.

9. Le dispositif de la revendication 1, **caractérisé par le fait que** les signaux de fonctionnement sont conçus pour empêcher une activation du système (3, 5, 6) de déplacement en vue d'une éjection de la préparation à travers l'orifice de sortie (2) lorsque l'orientation du dispositif est dans une première série de positions prédéterminées et inappropriées pour une administration et pour permettre une activation du système de déplacement en vue d'une éjection de la préparation à travers l'orifice de sortie lorsque l'orientation du dispositif est dans une seconde série de positions prédéterminées et appropriées pour une administration.

10. Le dispositif de la revendication 1, **caractérisé par le fait que** les messages sont une ou plusieurs alarmes ou instructions détectables par un opérateur, pour guider l'opérateur vers les actions indiquées.

11. Le dispositif de la revendication 1, **caractérisé par le fait que** le système (3, 5, 6) de déplacement est activé en actionnant le dispositif (6) et qu'un ou plusieurs signaux de fonctionnement sont un ou plusieurs signaux de commande pour le système d'actionnement.

12. Le dispositif de la revendication 11, **caractérisé par le fait que** le système d'actionnement comprend un dispositif doté d'un moteur électrique (6) pour le système (3, 5) de déplacement.

13. Le dispositif de la revendication 1, **caractérisé par le fait que** le système (3, 5, 6) de déplacement est doté d'un dispositif d'armement, d'un dispositif de vannes ou d'un dispositif de commande de fluide et que le ou les signaux de mise en fonctionnement sont un ou plusieurs signaux de commande pour ces dispositifs.

14. Le dispositif de n'importe laquelle des revendication de 1 à 13, **caractérisé par le fait que** l'unité de commande (7) est agencée pour permettre l'activation du système de déplacement (3, 5, 6) avec le dispositif dans n'importe quelle direction durant l'injection qui fait suite à l'activation du système de déplacement (3, 5, 6) en vue de la mise en état opérationnel.

15. Un procédé pour le fonctionnement d'un dispositif d'injection en vue de l'administration d'une ou de plusieurs injections au départ d'une cartouche (1) disposée dans un boîtier (9) et est muni à son extrémité arrière d'un piston (3) qui, par l'intermédiaire d'une tige de piston (5), peut être déplacé vers l'avant et dans lequel la mise en état opérationnel du dispositif, pour l'administration, après l'installation de ladite cartouche et l'administration ultérieure à partir de celle-ci, est commandée par une unité de commande électronique (7) comportant un détecteur, **caractérisé par le fait que** le détecteur est un détecteur de position (8) et que le procédé comporte les étapes a) d'obtention de la part du détecteur d'un signal indicatif de l'orientation du dispositif, b) de transfert du signal à l'unité de commande (7) et c) de mise en fonctionnement de l'unité de commande d'une manière telle que, durant l'opération de mise en état opérationnel du dispositif, un mouvement vers l'avant de la tige (5) du piston n'est rendu possible que lorsque l'axe longitudinal de la cartouche d'injection (1) est orientée dans une direction prédéterminée.

16. Le procédé suivant la revendication 15, **caractérisée par le fait que** l'étape de mise en mouvement de la tige (5) du piston est effectuée au moyen d'un moteur électrique (6), qui est commandé par ladite unité de commande (7).

17. Le procédé suivant la revendication 15, **caractérisée par le fait que** ladite étape de mise en état opérationnel du dispositif comprend l'étape d'évacuation du gaz hors de ladite cartouche (1) d'injection.

18. Le procédé suivant la revendication 15, **caractérisée par le fait que** ladite cartouche est une cartouche à compartiment multiple et **par le fait que** ladite étape de mise en état opérationnel comprend aussi une étape de reconstitution de la composition (3) à injecter.

19. Le procédé suivant la revendication 18, **caractérisée par le fait que** ladite étape de mise en état opérationnel du dispositif n'est rendue possible qu'avec l'axe longitudinal de la cartouche (1) dans une orientation essentiellement verticale avec son extrémité avant, à partir de laquelle a lieu l'administration, orientée vers le haut.

20. Le procédé suivant la revendication 18, **caractérisée par le fait que** ladite étape de mise en état opérationnel du dispositif n'est rendue possible qu'avec l'axe longitudinal de la cartouche (1) dans une orientation essentiellement verticale avec l'extrémité avant de la cartouche orientée vers le bas.

21. Le procédé suivant la revendication 15, **caractérisée par le fait que** l'étape d'actionnement comprend l'étape d'enregistrement d'un changement dans les signaux de position du détecteur (8).

22. Le procédé suivant la revendication 21, **caractérisée par le fait que** l'étape d'actionnement comprend de plus l'étape consistant à empêcher une administration à moins que le dispositif n'ait été tourné d'une manière prédéterminée.

23. Le procédé suivant la revendication 21, **caractérisée par le fait que** l'étape d'actionnement comprend de plus l'étape consistant à empêcher une administration répétée à moins que le dispositif n'ait été tourné d'une manière prédéterminée entre les administrations successives.

24. Le procédé suivant la revendication 15, **caractérisée par le fait que** l'étape d'actionnement comprend de plus l'étape consistant à empêcher une administration lorsque l'orientation du dispositif est dans une première série de positions prédéterminées et inappropriées pour une administration et permettre l'administration lorsque l'orientation du dispositif est dans une seconde série de positions prédéterminées et appropriées pour une administration.

25. Le procédé suivant la revendication 15, **caractérisée par le fait que** l'étape d'actionnement comprend l'étape d'émission d'un ou de plusieurs messages et/ou d'accomplissement d'un ou de plusieurs déplacements du piston (3) vers l'avant.

26. Le procédé suivant la revendication 25, **caractérisée par le fait que** les messages émis sont une ou plusieurs alarmes ou instructions détectables par un opérateur, en vue de la conduite de l'opérateur dans les actions indiquées.

27. Le procédé suivant la revendication 25, **caractérisée par le fait que** le dispositif comporte un système (6) d'actionnement et que les déplacements sont effectués par activation du système d'actionnement.

28. Le procédé suivant la revendication 25, **caractérisée par le fait que** le système d'actionnement est un système doté d'un moteur électrique (6)

29. Le procédé suivant la revendication 25, **caractérisée par** l'étape permettant l'administration dans n'importe quelle orientation après l'étape de mise en état opérationnel.

30. Le procédé suivant n'importe laquelle des revendications de 15 à 29, **caractérisée par le fait que**, après l'étape de mise en état opérationnel, une administration s'effectue par un nouveau déplacement du piston (3).
